(19) Europäisches Patentamt
European Patent Office
Office européen des brevets

(11) EP 3 115 017 B1

(12) **EUROPEAN PATENT SPECIFICATION**

(45) Date of publication and mention of the grant of the patent:
**03.07.2019 Bulletin 2019/27**

(21) Application number: **15758811.2**

(22) Date of filing: **02.03.2015**

(51) Int Cl.:
***A61C 5/00*** *(2017.01)* ***A61K 6/00*** *(2006.01)*

(86) International application number:
**PCT/JP2015/056039**

(87) International publication number:
**WO 2015/133418 (11.09.2015 Gazette 2015/36)**

# (54) PLASTIC CONTAINER FOR DENTAL ADHESIVE COMPOSITIONS

KUNSTSTOFFBEHÄLTER FÜR ZAHNÄRZTLICHE HAFTZUSAMMENSETZUNGEN

RÉCIPIENT EN PLASTIQUE POUR COMPOSITIONS ADHÉSIVES DENTAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.03.2014 JP 2014041534**

(43) Date of publication of application:
**11.01.2017 Bulletin 2017/02**

(73) Proprietor: **Tokuyama Dental Corporation**
**Tokyo 110-0016 (JP)**

(72) Inventors:
- **YAMASHITA, Yoshinobu**
  **Tokyo 110-0016 (JP)**
- **HIRATA, Kouichirou**
  **Tokyo 110-0016 (JP)**
- **YAMAGUCHI, Nobutoshi**
  **Tokyo 110-0016 (JP)**
- **KIMURA, Mikio**
  **Tokyo 110-0016 (JP)**

(74) Representative: **J A Kemp**
**14 South Square**
**Gray's Inn**
**London WC1R 5JJ (GB)**

(56) References cited:
**EP-A1- 2 615 043** **EP-A2- 0 669 113**
**WO-A1-2011/132714** **WO-A1-2012/033037**
**WO-A2-98/00071** **JP-A- H07 145 017**
**JP-A- H07 255 748** **JP-A- H11 206 812**
**JP-A- H11 349 427** **JP-A- 2000 513 968**
**JP-B2- 5 318 509** **JP-B2- H0 667 812**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Technical Field:

This invention relates to a plastic container for containing a dental adhesive composition. More specifically, the invention contains a plastic container for containing a dental adhesive composition, the plastic container being equipped with a knock button-type piston that moves each time when the knock button is depressed to eject the dental adhesive composition in a predetermined amount.

Background Art:

In the field of dental treatment, a restorative material called resin composite has heretofore been widely used for restoring a cavity of a tooth formed by the act of treating a decayed tooth. The resin composite is used for restoring the cavity formed by the decay of a relatively small degree. Namely, the resin composite is filled in the cavity, cured by being polymerized and is firmly fixed being buried in the cavity; i.e., the cavity is repaired. In addition to the resin composite, there has also been known another restorative material called, for example, resin cement. The resin cement, too, is filled in a cavity of a tooth. Here, however, the resin cement is chiefly used for fixing, to the tooth, a restoration (prosthesis) such as an inlay prepared outside the mouth. The resin cement is, in many cases, used when the tooth is greatly missing due to decay.

Here, adhesive property of the restorative material, such as resin composite or resin cement, to the teeth is not enough. Usually, therefore, an adhesive composition called dental adhesive or dental pretreating material is applied to the tooth surface in the cavity and is, as required, irradiated with light so as to be cured. Thereafter, the restorative material is filled therein.

The dental adhesive or the dental pretreating material, when it is to be used for the teeth, comprises an adhesive composition containing an acid component and water and, further, contains a polymerizable monomer, a polymerization initiator, solvents and other additives, as required.

The dental adhesive composition is applied into a cavity of a tooth formed by the act of treating the decay, and is used in a very small amount in one time. As a container for containing such a dental adhesive composition, therefore, the present applicant has proposed in a patent document 1 an ejection container of the knock-button type.

The ejection container of the knock-button type includes a container body of a hollow cylindrical shape and an ejection nozzle provided at an end of the container body on one side thereof. In the container body, a knock-button type piston member is fitted from an end of the container body on the other side thereof in a manner that the piston member can be depressed. Upon depressing the piston member, the dental adhesive composition contained in the container body is ejected though the ejection nozzle. Besides, the ejection nozzle has a very small inner diameter and when the knock button is depressed, therefore, the dental adhesive composition is constantly ejected in a predetermined very small amount from the ejection nozzle.

With the container of the patent document 1, each time when the knock button is depressed once, the dental adhesive composition is constantly ejected in a small predetermined amount and is applied into the cavity. Namely, the dental adhesive composition can be stably picked up in a small amount irrespective of the skill of a dentist offering a great advantage from the standpoint of cost.

However, even the container of the patent document 1 involves a problem with regard to storage stability of the dental adhesive composition. That is, if the dental adhesive composition contains a polymerizable monomer, the dental adhesive composition contained in the container undergoes the gelation while it is being stored in the container. To prevent the gelation, means is employed, such as storing the container in a refrigerator to maintain the composition in the container at a temperature considerably lower than room temperature. Still, however, it is difficult to effectively prevent the gelation. Specifically, with the container of the patent document 1 having a structure that permits the composition to be constantly ejected in a very small predetermined amount through a very thin ejection nozzle, even a slight degree of gelation of the composition could cause a great problem such as clogging in the ejection nozzle or a variation in the amount of ejection.

To alleviate the above problem, the gelation may be prevented by adding a polymerization inhibitor still, however, permitting the gelation to take place during the storage. If the polymerization inhibitor is added in large amounts, however, the dental adhesive composition often fails to exhibit its properties when it is used.

Moreover, when the container of the patent document 1 is stored in a refrigerator from the standpoint of storage stability as described above, the temperature of the container is lower than room temperature right after it is taken out from the refrigerator. The container is then gripped by hand and its content is heated. In this case, the content solution often tends to be ejected in amounts larger than what it would have been determined by the number of times of depressing the knock button.

Prior Art Document:

Patent Document:

Patent document 1: JP-A-2011-213415

EP 0 669 113 A2 and WO 98/00071 A2 also disclose plastic containers for containing a dental adhesive composition.

Outline of the Invention:

Problems that the Invention is to Solve:

It is, therefore, an object of the present invention to provide a plastic container for containing and ejecting a dental adhesive composition that contains a polymerizable monomer and a polymerization initiator, effectively preventing the gelation of the dental adhesive composition while it is being stored in the container and, further, improving the ejection performance.

Means for Solving the Problems:

According to the present invention, there is provided a A plastic container (1) for containing a dental adhesive composition that contains a polymerizable monomer and a polymerization initiator; said plastic container including a container body (3) of a hollow cylindrical shape, an ejection nozzle (5) provided at an end of said container body on one side thereof and a cap (9) detachably fitted to the ejection nozzle, and having a structure in which a knock-button type piston member (7) comprising a knock button (11) is fitted in said container body from an end on the other side thereof in a manner that the knock-button type piston member can be depressed, and the composition contained in said container body is discharged through said ejection nozzle as said piston member is depressed; wherein said body wall has a double-wall structure including an inner circumferential wall (23b) and an outer circumferential wall (23a), and forming a heat-insulating space that is a pneumatic layer (27) between the inner circumferential wall and the outer circumferential wall; wherein said outer circumferential wall is at least partly thicker than said inner circumferential wall; and wherein the inner circumferential wall has an oxygen permeability in a range of 10 to 500 cc/m2 day atm in a portion of the container body where the composition is contained.

In the plastic container of the present invention, it is desired that:

(1) The container body is formed of an olefin resin; and

(2) The plastic container is used as a dental material kit having a voidage of not larger than 10% by volume relative to the volume of a portion that contains the dental adhesive composition in the plastic container.

Effects of the Invention:

The container of the invention is used for containing a dental adhesive composition that contains a polymerizable monomer and a polymerization initiator. The container has such a structure that the dental adhesive composition contained in the container body is ejected from the ejection nozzle as the knock-button type piston member provided in the container body is depressed by depressing a knock button in a state where the cap fitted to the ejection nozzle has been removed therefrom. Here, a great feature resides in that the container wall of the portion where the composition is contained has an oxygen permeability which is relatively as large as 10 to 500 cc/$m^2$ ·day·atm making it possible to effectively prevent the composition from undergoing the gelation. For example, as will be demonstrated in Examples appearing later, when the one-package type dental adhesive composition containing a polymerization initiator and a polymerizable monomer is contained in the container of the invention and is stored with the cap being closed in an atmosphere of 45°C, the dental adhesive composition contained therein exhibits the same appearance as the initial appearance even after the passage of five weeks; i.e., the gelation is effectively prevented from taking place. On the other hand, if the same experiment is conducted by using a container of Comparative Example of which the container wall having an oxygen permeability smaller than the above range specified by the present invention, the dental adhesive composition that is contained is obviously gelled after the passage of five weeks.

That is, in polymerizing the dental adhesive composition that contains the polymerizable monomer, it has been known that the polymerization is inhibited due to the polymer radicals that have generated and that bond to oxygen in the air bringing the polymerization into a termination. The present invention, however, effectively prevents the gelation of the dental adhesive composition by utilizing the inhibition of polymerization caused by oxygen and by permitting oxygen to permeate in large amounts into the portion where the dental adhesive composition is contained in the container.

By utilizing the inhibition of polymerization caused by oxygen, the storage stability of the dental adhesive composition can be easily improved by decreasing the thickness of the container. With the knock-button type container being formed in a decreased thickness, however, if the knock button is depressed in an attempt to eject the dental adhesive composition contained in the container body, then the container may undergo the deformation since it has a small strength. Therefore, the knock button may not be properly depressed and the dental adhesive composition may not be properly ejected. Namely, according to the present invention, the ejection container effectively prevents the gelation of the dental adhesive composition despite the container is of the knock-button type.

According to the present invention as described above, even when the dental adhesive composition as represented by a dental adhesive or a dental pretreating material is contained in the container and is stored therein, it is made possible to effectively prevent the gelation of the dental adhesive composition, to effectively avoid the clogging in the ejection nozzle caused by the gelation and, besides, to effectively prevent a decrease in the properties of the dental adhesive composition caused by gelation.

Brief Description of the Drawing:

[Fig. 1] is a schematic side view illustrating, partly in cross section, the most preferred embodiment of a plastic container of the present invention.

Modes for Carrying Out the Invention:

<Structure of the container>

Referring to Fig. 1, a plastic container 1 generally designated at 1 includes a hollow cylindrical container body 3 and an ejection nozzle 5 formed at an end (tip) of the container body 3 on one side thereof. A knock-button type piston member 7 is provided in the container body 3.

A cap 9 is detachably fitted to the ejection nozzle 5.

The knock-button type piston member 7 is provided in the hollow cylindrical container body 3, and includes a piston rod 10, a knock button 11 for pushing the piston rod 10 toward the ejection nozzle 5, and a pusher 13 attached to the tip of the piston rod 10. The pusher 13 is fitted in the container body 3 and slides in the container body 1 toward the ejection nozzle 5 accompanying the operation of the piston rod 10 that is depressed by the knock button 11.

Namely, the region where the pusher 13 moves in the container body 3 is a portion (also called container chamber 15) that contains the dental adhesive composition. As the pusher 13 slides, the dental adhesive composition contained in the container chamber 15 is ejected through the ejection nozzle 5.

The mechanism of engagement of the piston rod 10 with the knock button 11 has been known per se. as disclosed in, for example, JP-A-2000-83967. As the knock button 11 is depressed, the piston rod 10 and the pusher 13 are pushed in and, therefore, the dental adhesive composition is ejected through the ejection nozzle 5 in an amount corresponding thereto.

The ejection nozzle 5 has a shape which as a whole becomes narrow toward a tip. The tip 5a is thin like the tip of a pen, has a thin ejection port 5b formed therein, and has a spiral thread 5c formed on the outer surface at the root portion thereof so that the cap 9 can be detachably attached to the ejection nozzle 5 due to the screw-engagement. Namely, in a state where the cap 9 is removed, the knock button 11 is depressed, and the dental adhesive composition contained in the container chamber 15 is ejected. The dental adhesive composition pushed out from the container chamber 15 is then ejected to the exterior through the ejection port 5b.

The ejection nozzle 5 of the form described above has the tip 5a that is as thin as the tip of a pen and, therefore, enables the dental adhesive composition to be picked up in a minimum required amount.

The ejection nozzle 5, further, has a reservoir space 20 formed in the root portion thereof. Namely, the dental adhesive composition pushed out from the container chamber 15 passes through the reservoir space 20, and is ejected through the ejection port 5b. Formation of the reservoir space 20 prevents such an inconvenience that, when the cap 9 is removed, the dental adhesive composition drips from the ejection port 5b.

In the plastic container 1, though not limited thereto only, it is desired that the body wall 23 of the hollow cylindrical container body 3 has a double-wall structure including an inner circumferential wall 23b and an outer circumferential wall 23a. A pneumatic layer 27 that serves as the heat-insulating layer is formed between the inner circumferential wall 23b and the outer circumferential wall 23a. That is, the plastic container 1 of the invention contains the dental adhesive composition that will be described later, the dental adhesive composition often containing a polymerizable monomer and a polymerization initiator. In this case, the plastic container 1 containing the dental adhesive composition is maintained at a low temperature being stored in a refrigerator or the like, and is taken out from the refrigerator when it is to be used. At the time of use, the cap 9 is removed, and the knock button 11 is depressed to eject the dental adhesive composition onto, for example, a pick-up dish and the like. Thereafter, by using a sponge or the like, the dental adhesive composition is applied onto a cavity in a tooth of a patient.

The body wall 23, on the other hand, may comprise an ordinary single wall. In this case, too, the plastic container 1 is taken out from the refrigerator and the cap 9 is removed. Here, however, a gas in the plastic container may expand being heated by the hand gripping the body wall 23 and cause such an inconvenience that the content readily drips down from the ejection port 5b before the tip 5a of the ejection nozzle 5 is placed on the pick-up dish and the like. On the other hand, upon forming the body wall 23 in the double-wall structure including the inner circumferential wall 23b and the outer circumferential wall 23a, there is formed the pneumatic layer 27 therebetween effectively alleviating such an inconvenience that the content expands due to the heat of the hand when the body wall 23 (outer circumferential wall 23a) is gripped by hand.

In forming the body wall 23 in the double-wall structure as described above, Fig. 1 illustrates an example in which the body wall 23 as a whole is assuming the double-wall structure. It is, however, also allowable to employ the double-wall structure in only the portion where the container chamber 15 is formed with the pusher 13 at the initial position.

The pneumatic layer is formed in space between the outer circumferential wall 23a and the inner circumferential wall 23b, and heat-insulating property of a suitable degree is obtained. To form the pneumatic layer, the air is introduced through, for example, a portion that is joining the outer circumferential wall 23a and the inner circumferential wall 23b together. The thickness of the pneumatic layer (i.e., gap between the outer circumferential wall 23a and the inner circumferential wall 23b) is, usually, about 0.1 to about 5 mm and, specifically, about 0.3 to about 3 mm.

The container body 3 of the plastic container 1 is formed relying on a forming means such as injection-forming means by using various thermoplastic resins that can be formed. After the container body 3 is formed, the knock-button type piston member 7 is fitted, the content is filled, the separately formed ejection nozzle 5 is fitted, and the separately formed cap 9 is fitted.

In the plastic container 1 of the present invention fabricated as described above, it is very important that the body wall 23 (inner circumference 23b in the example of Fig. 1) forming the container chamber 15 that contains the dental adhesive composition, has an oxygen permeability in a range of 10 to 500 cc/$m^2$·day·atm and, specifically, 30 to 300 cc/$m^2$·day· atm.

Namely, the dental adhesive composition that contains the polymerizable monomer and the polymerization initiator tends to be gelled while it is being stored. To prevent the gelation, the container 1 is stored in a refrigerator to maintain the content at a low temperature. Despite of employing this means, however, the gelation cannot be completely prevented. The gelation causes the clogging in the ejection port 5b in the ejection nozzle 5 and a decrease in the properties of the content (e.g., decrease in the adhesiveness to the teeth).

According to the present invention, on the other hand, the oxygen permeability is set as described above so that the polymerization is inhibited with oxygen that permeates into the container chamber 15 making it, therefore, possible to effectively prevent the gelation. In the conventional bottle-like containers, in particular, it could be expected to inhibit the polymerization with oxygen by filling the dental adhesive composition in an amount relatively smaller than the volume of the bottle. With the knock-button type plastic container of the present invention, however, the knock mechanism, too, must be contained in the container which is relatively thin and short, and the volume of the container chamber 15 tends to become relatively small. Therefore, the effect could not be so much exhibited for inhibiting the polymerization with oxygen.

If, for example, the oxygen permeability is smaller than the above range, oxygen is not sufficiently supplied into the container chamber 15, polymerization is not inhibited with oxygen, and the content cannot be prevented from gelling. Further, if the thickness of the body wall 23 (inner circumferential wall 23b in Fig. 1) is very decreased in order that the oxygen permeability exceeds the above range, then the container chamber 15 loses dimensional stability. Besides, the content cannot be property extruded by the pusher also accompanied by a problem of volatilization of volatile components such as solvent and water contained in the dental adhesive composition.

The oxygen permeability is set to lie within the above-mentioned range in order to feed oxygen in suitable amounts into the container chamber 15 and, therefore, to inhibit the polymerization with oxygen. When the double-wall structure shown in Fig. 1 is employed, the inner circumferential wall 23b forming the container chamber 15 placing the pusher 13 at least at the initial position, should have an oxygen permeability that lies within the above range. The same also holds true when the body wall 23 has the single-wall structure; i.e., the oxygen permeability should lie in the above range in the portion where the container chamber 15 is formed with the pusher 13 at least a the initial position.

To set the oxygen permeability to lie in the above range, the thickness of the body wall 23 (inner circumferential wall 23b in Fig. 1) forming the container chamber 15 should be selected depending on the kind of the resin forming the container 1.

For instance, a film of a predetermined thickness is formed by using the resin that forms the container 1. The film is then measured for its oxygen permeability from which a thickness is calculated such that the oxygen permeability lies within the above range. Thereafter, the container may be so formed as to assume the above thickness.

The oxygen permeability of the film can be measured by using, for example, an oxygen permeability measuring apparatus (OX-TRAN manufactured by Modern Controls, Inc.).

As the material for forming the container 1 of the present invention, there can be used various resins that are

formable as described above. From the standpoint of setting the oxygen permeability to lie within the above range, however, it is desired that the container of the invention is formed by using olefin resins, as exemplified by low-density polyethylene, linear low-density polyethylene, intermediate- or high-density polyethylene, polypropylene, poly(1-butene) or poly(4-methyl-1-pentne) and random or block copolymers of $\alpha$-olefins, such as ethylene, propylene, 1-butene or 4-methyl-1-petene and, in addition, cyclic olefin copolymers disclosed in JP-A-2007-284066. That is, these olefin resins have oxygen permeabilities that are larger than those of other resins (such as polyethylene terephthalate (PET) and polyvinyl chloride), and, therefore, enable the body wall 23 to assume a relatively large thickness yet permitting the oxygen permeabilities to lie within the above range. PET and the like resins, however, have low oxygen permeabilities. To bring the oxygen permeability to lie within the above range, therefore, the thickness must be very decreased causing, therefore, the body wall 23 to lose its strength.

In the present invention, as described above, it is desired to form the container 1 (body wall 23) by using an olefin resin having a large oxygen permeability. Despite of using the olefin resin, however, the thickness of the body wall 23 must be decreased to some extent if it is attempted to bring the oxygen permeability to lie in the above range. Therefore, some degree of decrease in the strength of the body wall 23 is not avoidable.

To alleviate the decrease in the strength according to the present invention, the body wall 23 is formed in a double-wall structure including the inner circumferential wall 23b and the outer circumferential wall 23a as shown in Fig. 1. Here, it is desired that the outer circumferential wall 23a has, at least partly or desirably entirely, a thickness larger than that of the inner circumferential wall 23b. If a gap is formed between the outer circumferential wall 23a and the knock button 11, the air in the pneumatic layer 27 is replaced by the external air. Therefore, the outer circumferential wall 23a does not affect the gelation of the content.

In the double-layer structure of Fig. 1, the outer circumferential wall 23a has, at least partly, a thickness larger than that of the inner circumferential wall 23b so that, even in case an external force is given thereto, the container is prevented from breaking or the content is prevented from leaking due to the deformation of the container. It is desired that the outer circumferential wall 23a has a polygonal shape in cross section and the corner portions have a large thickness. By forming the outer circumferential wall 23a in a polygonal shape in cross section, the container can be prevented from rolling when it is fallen. Further, by forming the corners in a large thickness, the content can be effectively prevented from leaking caused by deformation and the like of the container due to application of an external force thereto.

<Dental adhesive composition contained in the container chamber 15>

The plastic container of the invention having the structure as described above is used for containing the dental adhesive composition.

The composition, so far as it contains the polymerizable monomer and the polymerization initiator, may be, for example, a teeth pretreatment agent further containing an acid component and water, a prosthetic pretreatment agent further containing a coupling agent and a sulfur compound, or a teeth adhesive containing an acid group-containing polymerizable monomer as the polymerizable monomer.

The dental adhesive composition that contains the polymerizable monomer as a basic component has a problem in that it undergoes the gelation while it is stored in the container. By using the container of the invention described above, however, oxygen is suitably fed to the dental adhesive composition in the container. Therefore, the polymerization is inhibited with oxygen, and the dental adhesive composition is prevented from gelling.

1. Polymerizable monomers;

As the polymerizable monomer, a monomer that is typically used for this kind of dental adhesive composition can be used, and can be exemplified by monomers having, as a polymerizable group, a (meth)acryloyl group, a derivative group of (meth)acryloyl group such as (meth)acryloyloxy group, (meth)acryloylamino group or (meth)acryloylthio group, or vinyl group, allyl group or styryl group. Concretely, there can be exemplified the following mono(meth)acrylate monomers and polyfunctional (meth)acrylate monomers.

Mono(meth)acrylate monomers;

Methyl (meth)acrylate,
Ethyl (meth)acrylate,
Glycidyl (meth)acrylate,
2-Cyanomethyl (meth)acrylate,
Benzyl (meth)acrylate,
Polyethylene glycol mono(meth)acrylate,
Allyl (meth)acrylate,
2-Hydroxyethyl (meth)acrylate,

Glycidyl (meth)acrylate,
3-Hydroxypropyl (meth)acrylate,
Glyceryl mono(meth)acrylate, and
2-(Meth)acyloxyethylacetyl acetate.

Polyfunctional (meth)acrylate monomers;

Ethylene glycol di(meth)acrylate,
Diethylene glycol di(meth)acrylate,
Triethylene glycol di(meth)acrylate,
Nonaethylene glycol di(meth)acrylate,
Propylene glycol di(meth)acrylate,
Dipropylene glycol di(meth)acrylate,
2,2'-Bis[4-(meth)acryloyloxyethoxyphenyl] propane,
2,2'-Bis[4-(meth)acryloyloxyethoxyethoxyphenyl] propane,
2,2'-Bis{4-[3-(meth)acryloyloxy-2-hydroxypropoxy] phenyl} propane,
1,4-Butanediol di(meth)acrylate,
1,6-Hexanediol di(meth)acrylate,
Trimethylolpropane tri(meth)acrylate,
Urethane (meth)acrylate, and
Epoxy (meth)acrylate.

In addition to the above (meth) acrylate monomers, it is, further, allowable to use other polymerizable monomers, e.g., fumaric acid ester compounds such as dimethyl fumarate, diethyl fumarate and diphenyl fumarate; styrene derivatives such as styrene, divinylbenzene, $\alpha$-methylstyrene and $\alpha$ -methylstyrene dimer; and allyl compounds such as diallyl phthalate, dially terephthalate, diallyl carbonate and allyl diglycol carbonate.

The dental adhesive composition desirably contains a polymerizable monomer having an acid group (hereinafter referred to as acidic monomer). That is, the acidic monomer exhibits etching property to the teeth and, as a result, a large strength of adhesion is assured.

The acidic monomer is of such a nature that an aqueous solution or an aqueous suspension thereof exhibits acidity, and has at least an acid group and a radically polymerizable unsaturated group in a molecule thereof.

Representative examples of the acid group possessed by the acidic monomer include carboxyl group (-COOH) or an anhydride thereof, sulfo group ($-SO_3H$), phosphoric acid group {$-O-P(=O)(OH_2)$}, phosphinico group {=P(=O)OH}, and phosphono group {$-P(=O)(OH)_2$}. There is no specific limitation on the radically polymerizable unsaturated group, either, and any known one may be used. Concretely, there can be used (meth)acryloyl group, derivative groups of (meth)acryloyl group, such as (meth)acryloyloxy group, (meth)acryloylamino group and (meth)acryloylthio group, as well as vinyl group, allyl group and styryl group.

Use of the acidic monomers has been known. The present invention can use any one of those closely described in, for example, JP-A-2009-167132. Described below are the acidic monomers that can be used particularly preferably.

In the following compounds, $R_1$, $R_1$' and $R_1$" respectively represent a hydrogen atom or a methyl group.

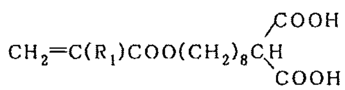

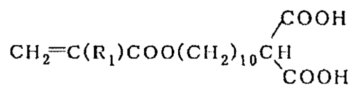

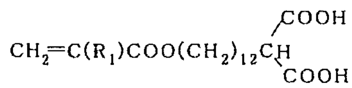

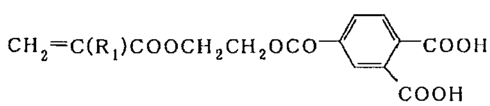

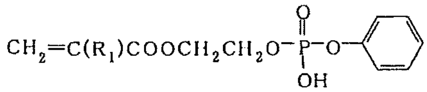

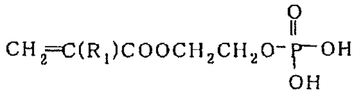

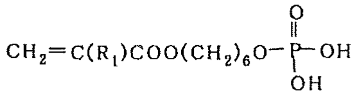

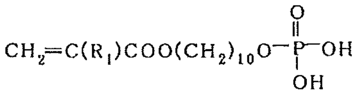

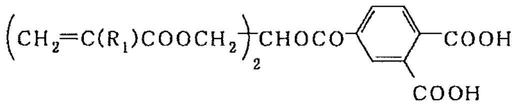

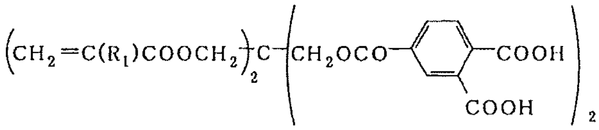

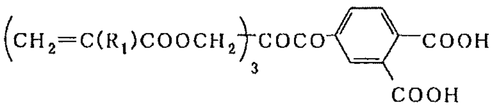

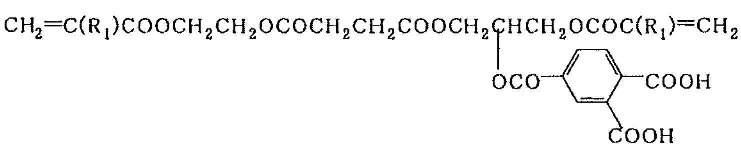

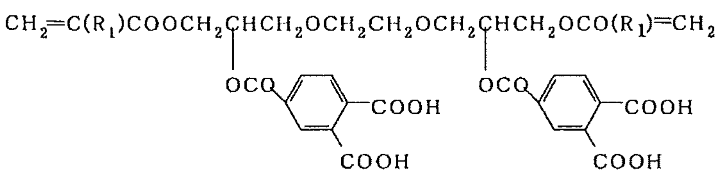

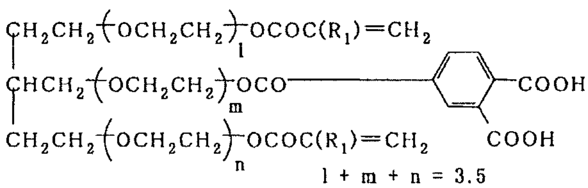

l + m + n = 3.5

$CH_2=C(R_1)CONHCH_2CH_2COOH$

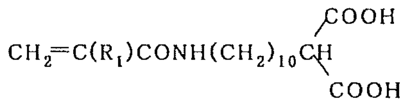

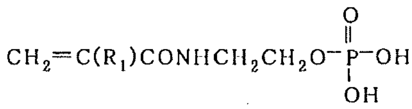

$CH_2=C(R_1)COOCH_2CH_2SO_3H$

$CH_2=C(R_1)COO(CH_2)_6SO_3H$

$CH_2=C(R_1)COO(CH_2)_{10}SO_3H$

$CH_2=C(R_1)COOC(CH_3)_2CH_2SO_3H$

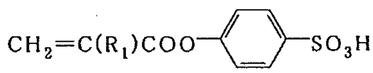

$CH_2=C(R_1)CONHCH_2CH_2SO_3H$

$CH_2=C(R_1)CONHC(CH_3)_2CH_2SO_3H$

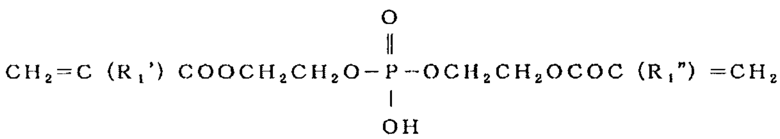

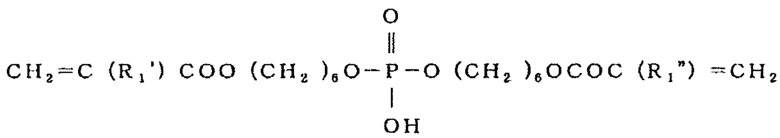

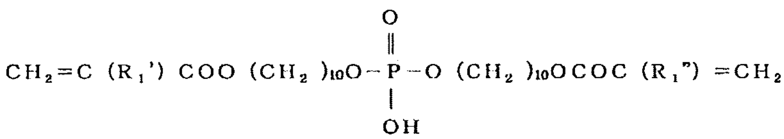

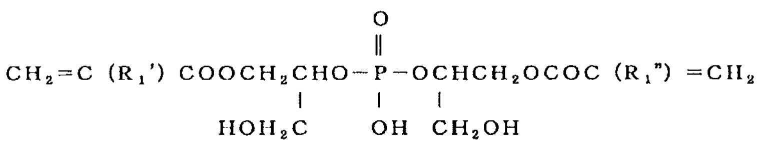

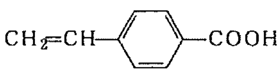

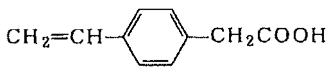

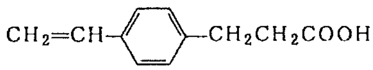

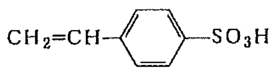

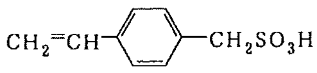

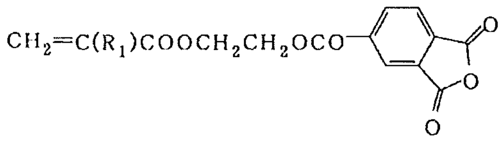

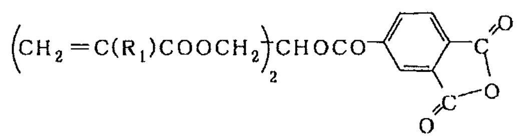

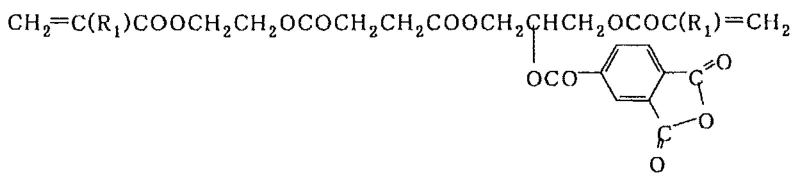

The above acidic monomers are used in a single kind or in a combination of two or more kinds. All of the radically polymerizable monomers do not have to be acidic monomers. Usually, the radically polymerizable monomers are used in combination with the generally used polymerizable monomers having no acid group.

The acidic monomer, for instance, is contained in an amount of not less than 5 parts by mass and, specifically, 10 to 80 parts by mass in 100 parts by mass of the whole radically polymerizable monomers. If the amount of the acidic monomer is small, etching property is not sufficiently exhibited to the teeth, and the strength of adhesion to the teeth is not satisfactory. Further, even if the acidic monomer is used in unnecessarily large amounts, the strength of adhesion does not increase beyond a certain level resulting, therefore, in an increase in the cost.

2. Polymerization initiators;

The polymerizable monomer mentioned above is formed into a cured product, for example, by intraoral polymerization after filling a cavity of a tooth therewith. When the dental adhesive composition of the invention is used as a dental adhesive or a dental pretreatment material, therefore, a polymerization initiator or a promotor is also often used to initiate the polymerization at a given point in time. The polymerization initiators can be divided into those of the chemical polymerization type and those of the photopolymerization type, and may be suitably selected depending on the object. It is, further, allowable to use the photopolymerization initiator and the chemical polymerization initiator in combination to obtain the adhesive composition of the dual cure type which can initiate both the photopolymerization and the chemical polymerization.

The photopolymerization initiator is favorably used for the dental adhesive that works to adhere a resin composite to the teeth and for the dental adhesive resin composite. Described below are representative examples of the photopolymerization initiator.

$\alpha$-Diketones such as diacetyl, acetylbenzoyl, benzil, 2,3-pentadione, 2,3-octadione, 4,4'-dimethoxybenzil, 4,4'-oxybenzil, camphorquinone, 9,10-phenanthrenequinone and acenaphthenequinone;
Benzoin alkyl ethers such as benzoin methyl ether, benzoin ethyl ether and benzoin propyl ether;
Thioxanthone derivatives such as 2,4-diethoxythioxanthone, 2-chlorothioxanthone and methylthioxanthone;
Benzophenone derivatives such as benzophenone, p,p'-dimethylaminobenzophenone and p,p'-methoxybenzophenone;
Acylphosphine oxide derivatives such as 2,4,6-trimethylbenzoyldiphenylphosphine oxide, bis(2,6-dimethoxybenzoyl)-2,4,4-trimethylpentylphosphine oxide, and bis(2,4,6-trimethylbenzoyl)phenylphosphine oxide; and
Those of the type of a combination of aryl borate compound, pigment and photo acid generator.

Among them, particularly preferred are a photopolymerization initiator of the type of $\alpha$-diketone, a photopolymerization initiator of the type of acylphosphine oxide, and a photopolymerization initiator of the type of a combination of aryl borate compound, pigment and photo acid generator.

As the $\alpha$-diketone, there is preferably used camphorquinone or benzil. As the acylphosphine oxide, there is preferably used 2,4,6-trimethylbenzoyldiphenylphosphine oxide, bis(2,6-dimethoxybenzoyl)-2,4,4-trimethylpentylphosphine oxide or bis(2,4,6-trimethylbenzoyl)phenylphosphine oxide.

The $\alpha$-diketone and acylphosphine oxide exhibit photopolymerizing activity even if they are used in a single kind. It is, however, desired that they are used in combination with an amine compound such as ethyl 4-dimethylaminobenzoate, lauryl 4-dimethylaminobenzoate or dimethylaminoethyl (meth)acrylate to attain higher polymerizing activity.

As the photopolymerization initiator of the type of a combination of aryl borate compound, pigment and photo acid generator, it is particularly desired to use the aryl borate compound such as sodium tetraphenylborate, to use, as the pigment, a coumarin-type pigment such as 3,3'-carbonylbis(7-diethylamino)coumarin or 3,3'-carbonylbis(4-cyano-7-diethylaminocoumarin), and to use, as the photo acid generator, a halomethyl group-substituted s-triazine derivative such as 2,4,6-tris(trichloromethyl)-s-triazine or a diphenyliodonium salt compound.

On the other hand, a chemical polymerization initiator or a promotor is preferably used for the material for pretreating the dental resin cement that is used for adhering a dental restorative material such as prosthesis to the teeth. The polymerization initiator of the type of chemical polymerization stands for the one that comprises a plurality of components which, when brought into contact, generate polymerization initiating species (radicals).

Concretely, there are used a system comprising an aryl borate compound and an acid compound; a system comprising an organic peroxide and an amine compound; a system comprising an azo compound and an organic peroxide; and a system comprising a pyrimidinetrione derivative, a halogen ion-forming compound and a metal ion-forming compound.

Among them, it is desired to use the system comprising the aryl borate compound and the acid compound, the system comprising the sulfinic acid (or sulfinic acid salt) and the acid compound, and the system comprising the organic peroxide and the amine compound, since they have high polymerizing activities and high levels of safety to the living body. Further, from such a standpoint that the composition containing the acid group-containing polymerizable monomer has a high polymerizing activity, it is desired to use the system comprising the aryl borate compound and the acid compound, and the system comprising the sulfinic acid (or sulfinic acid salt) and the acid compound. In the system comprising the aryl borate compound and the acid compound, further, it is desired to use, as the polymerization promotor, a metal compound having a valence of +II, +III, +IV or +V and, preferably, a vanadium compound having a valence of +VI and/or +V, which is a decomposition promotor for the organic peroxide, and/or an organic peroxide which is an oxidizing agent in combination.

As the aryl borate compound, there can be used any known one without limitation. Particularly preferred examples include amine salts or sodium salts of tetraphenylboric acid, tetra(p-fluorophenyl)boron, tetra(p-chlorophenyl)boron, trialkyl(p-fluorophenyl)boron, trialkyl(3,5-bistrifluoromethyl)phenylboron, dialkyldiphenylboron, and the like.

As the sulfinic acids (or sulfinic acid salt), there can be used the known ones without limitation. Particularly preferred examples include benzenesulfinic acid, o-toluenesulfinic acid, p-toluenesulfinic acid, ethylbenzenesulfinic acid, decylbenzenesulfinic acid, dodecylbenzenesulfinic acid, chlorobenzenesulfinic acid and naphthalenesulfinic acid which are aromatic sulfinic acids. Further, as the salts thereof, there can be used sodium benzenesulfinate, lithium benzenesulfinate, sodium p-toluenesulfinate, lithium p-toluenesulfinate, potassium p-toluenesulfinate, sodium m-nitrobenzenesulfinate, sodium p-fluorobenzenesulfinate, and the like.

As the acid compounds, there can be used the acidic monomers described above without the need of using any other particular compounds. As the other acid compounds, there can be used any known inorganic acids and organic acids as long as an aqueous solution or an aqueous suspension, which is obtained by dissolution or suspension thereof, is acidic. Preferably, there can be used known acid compounds such as nitric acid, sulfuric acid, hydrochloric acid, phosphoric acid and acetic acid within ranges in which they do not inhibit the adhesiveness. Here, if there is added an acid compound having acidity stronger than that of the acid group-containing polymerizable monomer, it is probable that the adhesiveness may be adversely affected. It is, therefore, desired to use an acid compound having acidity weaker than that of the acid group-containing polymerizable monomer.

As the metal compound having a valence of +II, +III, +IV or +V used as the polymerization promotor in the system comprising the aryl borate compound and the acid compound, there can be used any known compound that is

not the above-mentioned polyvalent metal compound. Preferably, there can be used vanadium compound, iron compound, copper compound, molybdenum compound, manganese compound, cobalt compound, tungsten compound and tin compound. Concrete examples are vanadium compounds such as divanadium tetroxide (IV), vanadium oxide acetylacetonate (IV), vanadium oxobis(1-phenyl-1,3-butanedionate) (IV), bis(maltolato)oxovanadium (IV), vanadium pentoxide (V), sodium metavanadate (V) and ammonium metavanadate (V).

Similarly, as the organic oxide which is the polymerization promotor, there can be exemplified ketone peroxide, peroxyketal, hydroperoxide, diaryl peroxide, peroxy ester, diacyl peroxide, peroxydicarbonate, and the like. Specifically preferred are diacyl peroxides and hydroperoxides. Concretely, the diacyloxides include isobutyryl peroxide, 2,4-dichlorobenzoyl peroxide, 3,5,5-trimethylhexanoyl peroxide, octanoyl peroxide and lauroyl peroxides. The hydroperoxides include p-methane hydroperoxide, diisopropylbenzene peroxide, 1,1,3,3-tetramethylbutyl hydroperoxide and cumene hydroperoxides.

The polymerization initiators of the invention can be added in a single kind. As required, however, they are used in a plurality of kinds in combination. Their amount is, desirably, in a range of 0.01 to 10 parts by mass per 100 parts by mass of the polymerizable monomers. If the amount thereof is less than 0.01 part by weight, the curability of the adhesive tends to decrease. If the amount thereof exceeds 10 parts by weight, on the other hand, the strength of the cured body of the adhesive tends to decrease. The amount of addition of the polymerization initiator is, more preferably, in a range of 0.1 to 8 parts by mass.

3. Other additives;

Depending on the use, the dental adhesive composition contained in the plastic container of the present invention is blended with the additives that have been known per se being added to the above-mentioned base components.

For instance, there are added water and water-soluble organic solvents (e.g., ethanol, isopropyl alcohol, and the like). Namely, water heightens the etching capability of the acidic monomer to the teeth. Owing to the water-soluble organic solvent, the composition is applied into the cavity. Thereafter, the air is blown to quickly remove water, and the curing by polymerization quickly proceeds.

Further, a pH-adjusting agent such as citric acid can also be used to maintain the pH of the dental adhesive composition to lie in a predetermined range to thereby stably maintain the decalcification with the acidic monomer.

It is, further, allowable to improve the strength of the cured body, to improve the strength of adhesion and durability thereof by using a filler such as silica. Moreover, there can be used a polymerization inhibitor such as hydroquinone. There can be, further, suitably added a polyvalent ion-releasing filler such as fluorosilicate glass or a metal alkoxide to form ionic crossliking with the acidic monomer and hence to improve the strength of adhesion to the teeth. It is, further, allowable to suitably add polymerization inhibitor, tackifier, ultraviolet-ray absorber, dye, pigment, and the like.

In the adhesive composition of the present invention, the polymerizable monomers, polymerization initiator and other additives can be all contained in the plastic container of the invention. Here, if the polymerization initiator is to be blended with the above chemical polymerization initiator, it is recommended, by taking the storage stability into consideration,

that the polymerizable monomers, other additives and one component of the polymerization initiator are contained in the plastic container of the invention and the polymerization initiator is contained in any other container; i.e., the adhesive composition is provided in the form of two packages or more packages.

The dental adhesive composition contained in the plastic container of the invention can be picked up in a predetermined small amount by removing the cap 9 from the container 1, directing the tip of the ejection nozzle 5 to a pick-up dish or the like, and depressing the knock button 11. Here, the gelling is effectively prevented, a predetermined quality is maintained and, besides, the clogging is prevented. Through the operation of one time, therefore, the dental adhesive composition can be picked up in a predetermined amount each time. The dental adhesive composition is, thereafter, cured to form a cured body having excellent adhesiveness to the teeth.

The dental adhesive composition can be favorably used as a dental adhesive which is an adhesive for a resin composite. The dental adhesive composition, depending upon its composition, however, can also be used as a dental pretreatment material for the teeth to which the dental adhesive has not been applied yet. Further, the dental adhesive composition by itself can be used as a dental adhesive resin composite having good adhesiveness to the teeth. The dental adhesive composition can be, further, used as a dental resin cement for adhering a dental restorative material such as prosthesis to the teeth.

The dental adhesive composition has a viscosity of, desirably, 1 to 10,000 mPa·s. If the viscosity is lower than 1 mPa·s, the dental adhesive composition may often leak out from the ejection nozzle 5 during the storage. If the viscosity is higher than 10,000 mPa·s, on the other hand, then the dental adhesive composition tends to be little ejected at the time of use.

In the present invention, it is desired that the dental adhesive composition is contained in the plastic container at a filling ratio of not less than 90% by volume relative to the volume (initial volume) of the container chamber 15. Or,

in other words, it is desired that the voidage of the container chamber 15 is not more than 10% by volume and, more preferably, not more than 6% by volume. With the voidage in the container chamber 15 being not more than 10% by volume, the air is present in small amounts in the container chamber, and the dental adhesive composition is not blown out from the nozzle 5 even if the air therein is thermally expanded. If the amount of the air is small, the effect is not almost expected for inhibiting the polymerization with oxygen and, therefore, the probability of gelation increases. Despite the voidage is very small as described above, however, the body wall 23 according to the present invention has an oxygen permeability that lies in a range of 10 to 500 cc/m$^2$·day·atm making it possible to effectively prevent the gelation of the content. Therefore, the method of setting the voidage to be not more than 10% could become a particularly preferred embodiment enabling a preferred ejection capability and a storage stability to be attained at the same time.

## EXAMPLES

Excellent effects of the invention will now be described by way of the following Experimental Examples.

[Polymerizable monomers]

BisGMA: 2,2-bis(4-(2-hydroxy-3-methacryloxypropoxy)phenyl) propane
3G: triethylene glycol dimethacrylate
HEMA: 2-hydroxyethyl methacrylate

[Polymerizable monomer having acid group]

SPM: a mixture of 2-methacryloyloxyethyl dihydrogen phosphate and bis(2-methacryloyloxyethyl) hydrogen phosphate at a ratio of 2:1.

[Volatile water-soluble organic solvent]

IPA: isopropyl alcohol

[Polymerization initiators]

CQ: camphorquinone
DMBE: ethyl p-N,N-dimethylaminobenzoate

[Polymerization inhibitor]

BHT: dibutylhydroxytoluene

<Preparation of the dental adhesive composition>

30 Grams of BisGMA, 20 g of 3G, 25 g of HEMA, 25 g of SPM, 60 g of IPA, 10 g of water, 1g of CQ, 1 g of DMBE and 0.5 g of BHT were mixed together and were stirred until they became homogeneous to obtain a dental adhesive composition. By using the Ostwald viscometer, it was confirmed that the dental adhesive composition possessed a viscosity of 20 mPa·s.

<Preparation of the plastic container>

A hollow cylinder (outer circumferential wall 23a) of a propylene having a square shape in cross section and a thickness of 2.0 mm at the corners was formed and was used as a container body. The ejection nozzle 5 was fitted to the tip of the hollow cylinder, and the cap 9 was detachably fitted to the nozzle 5 to obtain a container base body.
In Examples and Comparative Examples, a hollow tube was inserted and fixed in the container body (hollow cylinder), the hollow tube serving as the inner circumferential wall 23b having a thickness of 0.3 to 1.2 mm and having a piston member 7 fitted therein in advance. Further, the ejection nozzle 5 was attached to the container body and the cap 9 was fitted thereto to obtain an empty container.
The hollow tube was of a circular shape in cross section having an outer diameter of 13 mm.
The gap between the hollow cylinder (outer circumferential wall 23a) and the hollow tube (inner circumferential wall 23b) inserted therein was set to be quite the same in all Examples and Comparative Examples.

<Testing the gelation during the storage>

The dental adhesive composition prepared above was contained in the hollow tube of the empty container to thereby obtain a dental material kit.
The dental adhesive composition was filled while removing the ejection nozzle 5 that was provided with the cap 9. After dental adhesive composition been filled, the ejection nozzle 5 was fitted again to obtain the dental material kit.
The kit was stored at 45°C for 5 weeks or 8 weeks to confirm the gelation with the eye. The evaluation was on the following basis.
○: No gelation was confirmed.
×: Gelation was confirmed.
A symbol "×" represents the case when the gelation was confirmed

<Residual amount of the dental adhesive composition>

An empty container having the knock-button type piston member 7 fitted therein but containing no dental adhesive composition was measured for its weight. The container was, further, measured for its weight after it has been filled with the dental adhesive composition to thereby find the weight (initial weight) of the dental adhesive composition contained in the dental material kit.
Next, the dental material kit filled with the above composition was stored at 45°C for 8 weeks and then the dental material kit was measured for its weight (weight after stored) .
From the weights measured above, the residual amount of the adhesive composition was calculated according to the following formula,

$$\text{Residual amount} = 100 \times \text{weight after stored/initial weight}$$

The larger the residual amount, the larger the amount of the volatile component held in the dental adhesive composition.
It has been confirmed that there was no change in the weight of the empty container before and after the storage.

<Testing the state of when opened>

The dental material kit containing the dental adhesive composition was stored in a refrigerator for 24 hours and was, thereafter, taken out into an environment of room temperature. Five minutes, thereafter, the cap 9 was removed to see if the composition blew out from the ejection nozzle 5. The test was repeated 10 times, and the state of when opened was evaluated on the following basis.
×: The composition blew out 4 to 10 times (poor state of opening)
Δ: The composition blew out 1 to 3 times.
○: The composition blew out 0 time (best state of opening)

<Example 1>

The hollow tube (inner circumferential wall 23b) was formed by using the polypropylene. The hollow tube possessed an oxygen permeability of 78 cc/$m^2$·day·atm.
The amount of the dental adhesive composition was adjusted, and the adhesive composition was filled in the hollow portion (hollow portion where the piston member is not entering in, and the content can be contained) of the hollow tube in a manner that the voidage was 5% to thereby obtain a dental material kit.
The dental material kit was tested for its gelation during the storage and the state of when opened. The results were as shown in Table 1.

<Examples 2 to 12 and Comparative Examples 1 to 3>

The kinds of the materials forming the hollow tubes and the thicknesses of the hollow tubes were so varied that the hollow tubes (inner circumferential walls) possessed oxygen permeabilities as shown in Table 1. Further, the amounts of the dental adhesive composition filled therein were adjusted such that the voidages were as shown in Table 1 to obtain the dental material kits in the same manner as in Example 1.
The thus obtained dental material kits were tested for their gelation during the storage and the state of when

opened. The results were as shown in Table 1.

Examples 1 to 12 have dealt with the kits using the containers of the present invention, and there was no change even after they were stored at 45°C for 5 weeks. Comparative Examples 1 and 2, on the other hand, have dealt with the kits using the inner circumferential walls having small oxygen permeabilities. Gelation was confirmed after they were stored at 45°C for 5 weeks.

In Examples 5, 7, 8, 12 and in Comparative Example 3, the hollow tubes (inner circumferential walls 23b) possessed different oxygen permeabilities, and the residual amounts of the contents were as good as 99% in Example 5, 97% in Example 7, 96% in Example 8 and 93% in Example 12. In Comparative Example 3, on the other hand, the residual amount of the content was as very low as 85%.

In Examples 1, 2 and 3, the voidages were 5%, 1% and 3%, respectively, and the states of when opened were good (○). In Examples 6 and 9, the voidages were 7% and 9%, respectively, and the states of when opened were on an allowable level (states of when opened were evaluated to be (Δ)).

In Example 10, the voidage was as high as 15% and the evaluation was high concerning the gelation. However, the state of when opened was evaluated to be poor (×).

Table 1

| | Oxygen permeability of inner wall cc/m$^2$·day·atm | Material of inner wall | Voidage % | Gelation at 45°C | | Residual amount % | State of when opened |
|---|---|---|---|---|---|---|---|
| | | | | After 5 weeks at 45°C | After 8 weeks at 45°C | | |
| Example 1 | 78 | PP | 5 | ○ | ○ | 99 | ○ |
| Example 2 | 78 | PP | 1 | ○ | ○ | 99 | ○ |
| Example 3 | 78 | PP | 3 | ○ | ○ | 99 | ○ |
| Example 4 | 38 | PP | 5 | ○ | ○ | 100 | ○ |
| Example 5 | 243 | LDPE | 5 | ○ | ○ | 96 | ○ |
| Example 6 | 78 | PP | 7 | ○ | ○ | 99 | Δ |
| Example 7 | 158 | LDPE | 7 | ○ | ○ | 97 | Δ |
| Example 8 | 243 | LDPE | 7 | ○ | ○ | 96 | Δ |
| Example 9 | 78 | PP | 9 | ○ | ○ | 99 | Δ |
| Example 10 | 78 | PP | 15 | ○ | ○ | 99 | × |
| Example 11 | 20 | PP | 5 | ○ | × | 100 | ○ |
| Example 12 | 425 | LDPE | 5 | ○ | ○ | 93 | ○ |
| Comp. Ex. 1 | 2.6 | PVC | 5 | × | × | 100 | ○ |
| Comp. Ex. 2 | 1.4 | PET | 5 | × | × | 100 | ○ |
| Comp. Ex. 3 | 867 | LDPE | 5 | ○ | ○ | 85 | ○ |

PP: polypropylene
LDPE: Low-density polyethylene
PVC: polyvinyl chloride

Description of Reference Numerals:

1: plastic container
3: container body
5: ejection nozzle
7: knock button-type piston member
9: cap
10: piston rod
11: knock button
13: pusher

## Claims

1. A plastic container (1) for containing a dental adhesive composition that contains a polymerizable monomer and a polymerization initiator;
said plastic container including a container body (3) of a hollow cylindrical shape, an ejection nozzle (5) provided at an end of said container body on one side thereof and a cap (9) detachably fitted to the ejection nozzle, and having a structure in which a knock-button type piston member (7) comprising a knock button (11) is fitted in said container body from an end on the other side thereof in a manner that the knock-button type piston member can be depressed, and the composition contained in said container body is discharged through said ejection nozzle as said piston member is depressed;
wherein said body wall has a double-wall structure including an inner circumferential wall (23b) and an outer circumferential wall (23a), and forming a heat-insulating space that is a pneumatic layer (27) between the inner circumferential wall and the outer circumferential wall;
wherein said outer circumferential wall is at least partly thicker than said inner circumferential wall;
and wherein the inner circumferential wall has an oxygen permeability in a range of 10 to 500 cc/$m^2$·day·atm in a portion of the container body where the composition is contained.

2. A plastic container according to claim 1, which comprises in the container body (3) a dental adhesive composition that contains a polymerizable monomer and a polymerization initiator.

3. The plastic container according to claim 1 or 2, wherein said container body (3) is formed of an olefin resin.

4. A dental material kit obtained by containing a dental adhesive composition in the plastic container (1) of claim 1, the dental adhesive composition containing a polymerizable monomer and a polymerization initiator, wherein the dental material kit has a voidage of not larger than 10% by volume relative to the volume of a portion that contains the dental adhesive composition in the plastic container.

## Patentansprüche

1. Kunststoffbehälter (1) zum Enthalten einer Dentalklebstoffzusammensetzung, die ein polymerisierbares Monomer und einen Polymerisationsinitiator enthält;
wobei der Kunststoffbehälter einen Behälterkörper (3) in einer hohlen, zylindrischen Form, eine Ausstoßdüse (5), die an einem Ende des Behälterkörpers auf einer Seite davon bereitgestellt ist, und eine Kappe (9), die lösbar an der Ausstoßdüse angebracht ist, beinhaltet und eine Struktur aufweist, bei der ein Kolbenelement vom Stoßknopftyp (7), umfassend einen Stoßknopf (11), in dem Behälterkörper von einem Ende auf der anderen Seite davon auf eine Weise angebracht ist, dass das Kolbenelement vom Stoßknopftyp heruntergedrückt werden kann, und die Zusammensetzung, die in dem Behälterkörper enthalten ist, durch die Ausstoßdüse ausgegeben wird, wenn das Kolbenelement heruntergedrückt ist;
wobei die Körperwand eine Doppelwandstruktur aufweist, die eine Innenumfangswand (23b) und eine Außenumfangswand (23a) beinhaltet und einen Wärmeisolierraum bildet, der eine pneumatische Schicht (27) zwischen der Innenumfangswand und der Außenumfangswand ist;
wobei die Außenumfangswand mindestens teilweise stärker als die Innenumfangswand ist;
und wobei die Innenumfangswand eine Sauerstoffdurchlässigkeit in einem Bereich von 10 bis 500 cc/$m^2$·Tag·atm

in einem Abschnitt des Behälterkörpers aufweist, in dem die Zusammensetzung enthalten ist.

**2.** Kunststoffbehälter nach Anspruch 1, der in dem Behälterkörper (3) eine Dentalklebstoffzusammensetzung umfasst, die ein polymerisierbares Monomer und einen Polymerisationsinitiator enthält.

**3.** Kunststoffbehälter nach Anspruch 1 oder 2, wobei der Behälterkörper (3) aus einem Olefinharz gebildet ist.

**4.** Dentalmaterialkit, das durch Enthalten einer Dentalklebstoffzusammensetzung in dem Kunststoffbehälter (1) nach Anspruch 1 erhalten wird, wobei die Dentalklebstoffzusammensetzung ein polymerisierbares Monomer und einen Polymerisationsinitiator enthält, wobei das Dentalmaterialkit eine Porosität von nicht größer als 10 Vol.-% relativ zum Volumen eines Abschnitts, der die Dentalklebstoffzusammensetzung in dem Kunststoffbehälter enthält, aufweist.

## Revendications

**1.** Contenant en plastique (1) destiné à contenir une composition d'adhésif dentaire qui contient un monomère polymérisable et un initiateur de polymérisation ;
ledit contenant en plastique comprenant un corps de contenant (3) de forme cylindrique creuse, une buse d'éjection (5) prévue à une extrémité dudit corps de contenant sur un côté de celui-ci et un capuchon (9) adapté de manière détachable sur la buse d'éjection, et ayant une structure dans laquelle un élément de piston de type à bouton à coup (7) comprenant un bouton à coup (11) est adapté dans ledit corps de contenant à partir d'une extrémité sur son autre côté de telle manière que l'élément de piston de type à bouton à coup puisse être enfoncé et que la composition contenue dans ledit corps de contenant soit déchargée à travers ladite buse d'éjection lorsque ledit élément de piston est enfoncé ;
ladite paroi de corps ayant une structure à paroi double comprenant une paroi circonférentielle interne (23b) et une paroi circonférentielle externe (23a), et formant un espace thermiquement isolant qui est une couche pneumatique (27) entre la paroi circonférentielle internet et la paroi circonférentielle externe ;
ladite paroi circonférentielle externe étant au moins partiellement plus épaisse que ladite paroi circonférentielle interne ;
et ladite paroi circonférentielle interne présentant une perméabilité à l'oxygène dans une plage de 10 à 500 $cm^3/m^2 \cdot$jour$\cdot$atm dans une partie du corps de contenant où est contenue la composition.

**2.** Contenant en plastique selon la revendication 1, qui comprend dans le corps de contenant (3) une composition d'adhésif dentaire qui contient un monomère polymérisable et un initiateur de polymérisation.

**3.** Contenant en plastique selon la revendication 1 ou 2, dans lequel ledit corps de contenant (3) est formé d'une résine d'oléfine.

**4.** Kit de matière dentaire obtenu par une composition d'adhésif dentaire contenue dans le contenant en plastique (1) de la revendication 1, la composition d'adhésif dentaire contenant un monomère polymérisable et un initiateur de polymérisation, le kit de matière dentaire ayant un volume de vide non supérieur à 10 % en volume par rapport au volume d'une partie qui contient la composition d'adhésif dentaire dans le contenant en plastique.

Fig. 1

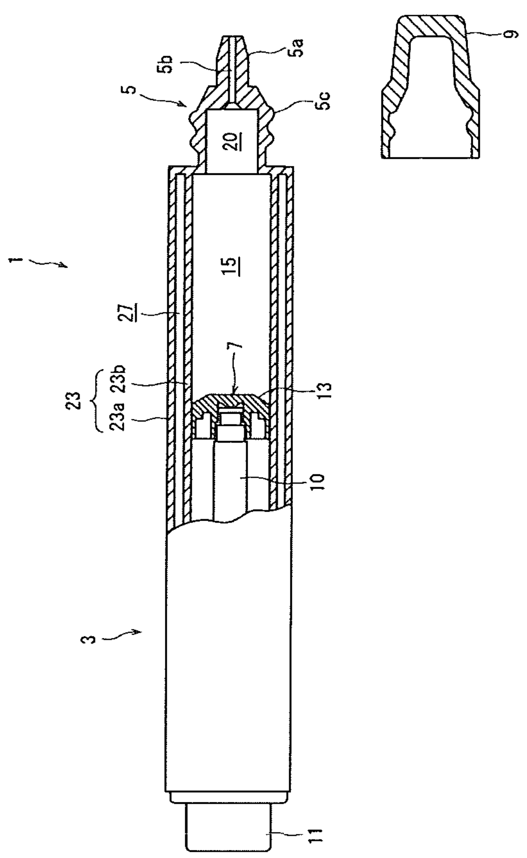

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011213415 A
- EP 0669113 A2
- WO 9800071 A2
- JP 2000083967 A
- JP 2007284066 A
- JP 2009167132 A